# FASCICULE DE BREVET EUROPEEN

(11) **EP 3 273 799 B1**
(45) Date de publication et mention de la délivrance du brevet: **12.05.2021**
(21) Numéro de dépôt: 16718409.2
(22) Date de dépôt: 24.03.2016
(51) Int. Cl.: A23L 2/84, A23F 3/16, C12N 1/06, C12H 1/052

(54) **UTILISATION D'UN EXTRAIT DE LEVURE POUR LE COLLAGE DE MOUTS ET DE BOISSONS**
VERWENDUNG VON HEFEEXTRAKT ZUR KLÄRUNG VON MOST UND GETRÄNKEN
USE OF A YEAST EXTRACT FOR CLARIFYING MUSTS AND BEVERAGES

(30) Priorité: 24.03.2015 FR 1552464
(43) Date de publication de la demande: 31.01.2018
(73) Titulaire: Lesaffre et Compagnie, 75001 Paris (FR)
(72) Inventeur: DORIGNAC, Etienne, 09270 Mazeres (FR); MENIN, Rudy, 94600 Choisy Le Roi (FR); GOSSELIN, Yves, 62157 Allouagne (FR)
(74) Mandataire: Plasseraud IP
(86) Numéro de dépôt international: PCT/FR2016/050660
(87) Numéro de publication internationale: WO 2016/151257

(56) Documents cités:
- EP-A1- 0 299 078
- EP-A1- 1 240 306
- EP-A1- 2 255 679
- EP-A1- 2 256 184
- EP-A1- 2 258 831
- WO-A1-01/46380
- WO-A1-96/13571
- WO-A1-03/063613
- WO-A1-03/063614
- WO-A1-2005/013723
- US-A1- 2003 162 735
- US-A1- 2010 178 300

## Description

La présente invention a pour objet une méthode de précipitation des particules visibles ou invisibles présentes en suspension dans un moût ou un liquide, comprenant l'utilisation d'un extrait de levure pour le collage de moûts et de liquides, en particulier de boissons telles que le vin, le thé, la bière ou les jus de fruits

WO0146380 décrit un procédé pour stabiliser le vin, qui comprend l'addition d'une préparation contenant des oligonucléotides ou des polynucléotides dans les moûts de raisin ou dans le vin. Ces oligonucléotides ou polynucléotides empêchent ou retardent la cristallisation des sels d'acide tartrique dans le vin, en prévenant à la fois le processus de nucléation et la croissance des cristaux des sels tartriques.

Le « collage » est une technique qui consiste à introduire dans un produit à traiter (liquides, moûts) une substance capable de floculer et de sédimenter en précipitant dans sa sédimentation les particules en suspension dans ledit produit, et ceci dans le but d'améliorer la limpidité, la filtrabilité, la stabilité ainsi que certaines qualités organoleptiques dudit produit.

Ainsi, de par le collage, les particules visibles et/ou invisibles en suspension dans le produit ainsi que la charge en colloïdes, qui sont responsables du trouble et du défaut de filtrabilité dudit produit, sont fortement réduits, voire totalement éliminés.

En ce qui concerne une boisson telle que le vin, le défaut de stabilité de ce dernier se traduit souvent par l'apparition de dépôts et/ou de troubles dus à la dégradation protéique ou au développement microbiologique. La diminution de la charge en particules instables par le collage permet de retarder ou de limiter l'apparition des dépôts et/ou des troubles.

Le collage peut améliorer certains aspects gustatifs des vins comme l'amertume ou l'astringence. Mais le collage, quand il est mal maîtrisé, peut aussi affaiblir la structure et l'intensité aromatique de certains vins.

Le collage peut aussi contribuer à gommer certains défauts ou déviations dus généralement à un mauvais état sanitaire.

Le collage des vins est connu et pratiqué depuis très longtemps. C'est au cours du XIXème siècle que sa pratique est devenue systématique, notamment sur des volumes importants de vins. Aujourd'hui, le collage se pratique même chez les producteurs de petits volumes.

Les produits de collages, désignés ci-après par « colles », utilisés couramment ces dernières années dans le domaine du vin sont la colle de poisson (qui est une protéine brute extraite des vessies natatoires de poissons), la caséine, le blanc d'œuf, les gélatines animales ou végétales, les bentonites (qui sont des argiles naturelles), la polyvinylpyrrolidone (« PVPP », qui est un produit de synthèse ayant une forte affinité pour les polyphénols). La colle PVPP est souvent associée à la caséine et permet de gommer l'astringence des vins rouges jeunes et l'amertume et la couleur jaune des vins blancs.

Les quatre premières colles citées présentent l'inconvénient d'être d'origine animale, et peuvent donc poser des problématiques allergènes. La PVPP a quant à elle une connotation chimique et présente une toxicité lors de sa manipulation.

Afin d'améliorer la qualité des colles pour les vins, des travaux de recherche ont porté sur des produits naturels, comme par exemple des levures. Une étude de 2006 (« Revue des œnologues, N° 120 ; pages 47-50, 2006) a ainsi montré qu'un extrait protéique de levure (désigné ci-après par « EPL ») a une action de collage équivalente, voire meilleure, que celle des colles utilisées classiquement.

Cependant, il existe toujours un besoin à ce jour de mettre au point d'autres colles issues de produits naturels, et présentant encore plus d'efficacité que les colles existantes, notamment dans le domaine du vin.

Ainsi un des buts de l'invention est de fournir aux producteurs et distributeurs de vins un produit naturel destiné à être utilisé dans les opérations de collage des moûts et des vins.

En ce qui concerne une boisson telle que le thé, il peut s'avérer nécessaire et intéressant de faire précipiter les particules visibles ou invisibles présentes dans le thé, et notamment les tanins.

En effet, un thé riche en tanins est aisément reconnaissable à l'astringence de sa liqueur, qui devient parfois de l'amertume lorsque le thé est trop infusé. Les tanins se libèrent lentement mais de façon croissante, ainsi une infusion trop longue augmente considérablement leur concentration et donne au thé son amertume lorsque le thé est trop infusé.

Par ailleurs les tanins présents dans le thé, même s'ils sont intéressants à plusieurs titres pour la santé, ont cependant un défaut : ils empêchent le fer des aliments d'être absorbé complètement par l'organisme lors de la digestion. Une consommation quotidienne importante de thé pourra donc avoir des conséquences sur l'assimilation du fer par l'organisme.

Cependant, la précipitation des tanins n'est pas forcément aisée à réaliser, et souvent plusieurs étapes de chromatographie sont nécessaires pour récupérer et purifier les tanins.

Un autre but de l'invention est donc de fournir un produit naturel destiné à être utilisé dans les opérations de collage d'une boisson telle que le thé, et plus particulièrement destiné à précipiter les tanins du thé.

Les travaux et recherches menés par les Inventeurs sur les applications œnologiques des levures et dérivés de levures ont permis de mettre au point un extrait de levure riche en acides nucléiques, et notamment en acides ribonucléiques, de taille supérieure à 10 KDa dont l'utilisation comme colle pour les produits de vinification est plus que prometteuse.

Par ailleurs, les recherches menées par les Inventeurs ont également permis de découvrir de manière surprenante que cet extrait de levure riche en acides nucléiques, et notamment en acides ribonucléiques, était particulièrement bien adapté comme colle pour les thés, et notamment pour faire précipiter les tanins du thé.

Cette découverte est d'autant plus inattendue étant donné que le document WO 2016/010064 décrit un extrait de levure comprenant au moins 50% d'ARN, pour empêcher le trouble du thé avant que ce dernier n'apparaisse, ledit trouble survenant fréquemment lors des conditions de stockage du thé.

En effet, contrairement à WO 2016/010064, l'extrait de levure riche en ARN n'est pas utilisé dans l'invention pour éviter l'apparition du trouble dans le thé, mais pour faire précipiter les particules visibles et invisibles du thé, et notamment les tanins.

Les Inventeurs ont également découvert de manière surprenante que cet extrait permettait de faire précipiter les pigments du thé.

La présente invention a plus particulièrement pour objet une méthode de précipitation des particules visibles ou invisibles présentes en suspension dans un moût ou un liquide, ladite méthode comprenant l'introduction d'un extrait de levure comprenant une quantité en poids d'acides nucléiques d'au moins 10%, de préférence d'au moins 15%, plus préférentiellement de 20 à 95% et encore plus préférentiellement de 30 à 48% par rapport au poids total dudit extrait, lesdits acides nucléiques étant formés de fragments d'acide ribonucléique (ARN) , dans ledit moût ou liquide, ledit moût ou liquide étant choisi parmi le vin, le thé, la bière ou les jus de fruits, la précipitation dudit extrait de levure avec les particules visibles ou invisible en suspension dans ledit moût ou liquide conduisant au collage dudit moût ou liquide.

Comme décrit précédemment, on entend par « collage », l'opération qui consiste à introduire dans le liquide à clarifier une substance capable de floculer et de précipiter, ladite substance, en floculant et en précipitant, entraînant dans sa chute toutes les particules en suspension présentes dans le liquide.

Selon l'invention le collage n'est pas destiné à prévenir ou empêcher le trouble du liquide, et notamment du vin ou du thé, avant qu'il ne se produise, mais au contraire à précipiter les particules visibles ou invisibles présentes dans ledit liquide.

Avantageusement selon l'invention, l'utilisation d'un extrait de levure riche en acides ribonucléiques permet notamment la précipitation des tanins du vin et du thé.

Tout comme la précipitation des tanins du thé, la précipitation des tanins du vin peut s'avérer intéressante dans certains cas.

En effet, les tanins du vin sont responsables de la sensation de râpeux en bouche, et assèchent la bouche.

On entend par « extrait de levure » les produits issus de la plasmolyse et lyse de levure intègre. On entend par levure « intègre » une levure « entière », vivante ou désactivée.

La levure présente des quantités importantes d'acide ribonucléique (ARN). En général on considère que quatre vingt dix neuf pour cent en masse des acides nucléiques d'une levure sont représentés par l'ARN. Cet ARN est constitué à 80% d'ARN ribosomal.

L'acide ribonucléique (ARN) est un acide nucléique, c'est-à-dire une molécule constituée d'un enchaînement de nucléotides.

Chaque nucléotide de l'ARN est constitué :
- d'un pentose, le ribose, dont les atomes de carbone sont numérotés de 1' à 5',
- d'une base nucléique ou base azotée (adénine « A », uracile « U », cytosine « C » et guanine « G »),
- d'un groupe phosphate.

La base azotée (A, U, C ou G) est reliée par un atome d'azote au carbone 1' du ribose. Les nucléotides sont liés les uns aux autres par des groupes phosphates, par l'intermédiaire de liaisons phosphodiester au niveau des carbones 3' et 5'.

Un extrait de levure tel que défini ci-dessus, comprenant notamment une quantité en poids d'acide ribonucléique d'au moins 10%, de préférence d'au moins 15%, plus préférentiellement de 20 à 95% et encore plus préférentiellement de 30 à 48%, par rapport au poids total dudit extrait, sera encore dénommé dans la présente demande « extrait nucléique de levure » (« ENL »).

Chacun desdits fragments d'ARN de l'extrait tel que défini ci-dessus et tel qu'utilisé selon l'invention présente une masse moléculaire moyenne supérieure à 10 kDa, de préférence allant de 30 à 110 kDa.

L' extrait nucléique de levure tel que défini ci-dessus et tel qu'utilisé selon l'invention comprend en outre des matières minérales, des saccharides (notamment des glucanes et des mannanes), et des acides aminés libres.

A titre d'exemple, dans un extrait nucléique de levure :
- les matières minérales sont présentes en une quantité en poids allant de 5 à 30%, de préférence de 15 à 25% par rapport au poids total de l'extrait de levure ;
- les mannanes sont présents en une quantité en poids allant de 5 à 25%, de préférence de 10 à 20% par rapport au poids total de l'extrait de levure ;
- les glucanes sont présents en une quantité en poids allant de 1 à 10%, de préférence de 3 à 8% par rapport au poids total de l'extrait de levure ;
- les acides aminés libres sont présents en une quantité en poids allant de 1 à 10%, de préférence de 3 à 8% par rapport au poids total de l'extrait de levure.

L' extrait de levure tel que défini ci-dessus et tel qu'utilisé selon l'invention pourra se présenter sous la forme d'une poudre ou d'un liquide plus ou moins concentré, et de préférence sous forme d'une poudre.

Selon l'invention la levure est par exemple choisie dans le groupe comprenant *Saccharomyces, Kluyveromyces, Torula, Candida,* et est de préférence *Saccharomyces,* et avantageusement *Saccharomyces cerevisiae.*

Le procédé de préparation de l'extrait de levure tel que défini ci-dessus et tel qu'utilisé selon l'invention permet avantageusement de préserver l'ARN naturellement présent dans la levure.

Le procédé de préparation d'un extrait de levure (ENL) tel que défini ci-dessus comprend les étapes suivantes :
- plasmolyse mécanique, physique, osmotique, thermique, enzymatique et/ou plasmolyse par modification de pH d'une levure intègre, afin d'une part de libérer les macromolécules internes de ladite levure dans leur état natif et d'autre part d'inactiver les enzymes de lyse de ces macromolécules,
- séparation par centrifugation,
- récupération de la fraction soluble contenant l'ENL,
- éventuellement, séchage de la fraction soluble.

La plasmolyse thermique sera effectuée à une température suffisante pour stopper, inhiber l'action des enzymes de lyse des macromolécules internes présentes dans la levure, et notamment des nucléases, ce qui permet avantageusement d'empêcher la lyse des chaînes nucléiques, et donc de limiter la formation de molécules de petites tailles moléculaires.

L'étape de plasmolyse thermique sera par exemple effectuée à une température de 60 à 120°C, de préférence de 70°C à 90°C, et plus préférentiellement encore à une température de 80°C, pendant une durée allant de 1 à 18 heures, de préférence de 5 à 15 heures, et plus préférentiellement encore de 11h.

La plasmolyse enzymatique ciblée permet avantageusement de libérer les macromolécules internes de levure sans les découper.

La plasmolyse par modification de pH permet la lyse de la levure et la libération de son contenu cellulaire.

Sans vouloir être liés par aucune théorie, les Inventeurs sont d'avis que la plasmolyse permet d'endommager la membrane plasmique et la paroi des cellules de levure au point de permettre des ouvertures suffisamment importantes pour laisser sortir des complexes nucléoprotéiques que sont les ribosomes.

Selon un mode de réalisation avantageux, l'invention a pour objet l'utilisation d'un extrait de levure tel que défini ci-dessus, pour le collage des vins.

Avantageusement selon l'invention, lorsque l'extrait nucléique de levure est utilisé pour le collage des moûts et des vins, il sera par exemple utilisé sous la forme d'une poudre. Ladite poudre sera par exemple utilisée à une teneur allant de 0,1 g (gramme) à 50 g par hectolitre (hl) de vin, et de préférence de 5 à 30 g/hl.

L'utilisation de doses appropriées de l'extrait nucléique de levure de l'invention permet une clarification rapide et une stabilité colloïdale du vin.

L'utilisation de doses excessives de l'extrait nucléique de levure de l'invention risque d'aboutir à un phénomène de « surcollage », c'est-à-dire la présence, après collage, de la colle non floculée. Le surcollage peut, dans des mauvaises conditions de conservation, générer un trouble.

Selon un autre mode de réalisation avantageux, l'invention a pour objet l'utilisation d'un extrait de levure tel que défini ci-dessus, pour le collage du thé, et en particulier pour la précipitation des tanins du thé et/ou des pigments du thé.

Avantageusement selon l'invention, lorsque l'extrait nucléique de levure est utilisé pour le collage des thés, il sera par exemple utilisé sous la forme d'une poudre. Ladite poudre sera par exemple utilisée à une teneur allant de 0,01 g (gramme) à 5 g par litre (1) de thé, et de préférence de 0,05 à 1 g/l.

L'utilisation d'un extrait de levure pour faire précipiter les pigments du thé permet ainsi de décolorer le thé, ce qui est recherché dans certains types de thé.

Les extraits nucléiques de levure tels que définis ci-dessus ont une durée de conservation au minimum de 3 ans en emballage non ouvert, stockés dans des locaux tempérés à l'abri de l'humidité.

L'invention a encore pour objet l'utilisation de fragments d'ARN provenant d'une levure choisie dans le groupe comprenant *Saccharomyces, Kluyveromyces, Torula, Candida* et leurs mélanges, pour le collage de moûts et de liquides, en particulier de boissons choisies parmi le vin, le thé, la bière ou les jus de fruits.

Selon un mode de réalisation avantageux de l'invention, les fragments d'ARN ci-dessus définis sont plus particulièrement utilisés pour précipiter les tanins et/ou pigments présents dans le thé.

La présente invention va maintenant être illustrée à l'aide des exemples et des figures qui suivent, qui sont donnés à titre d'illustration et ne donc sont nullement limitatifs.

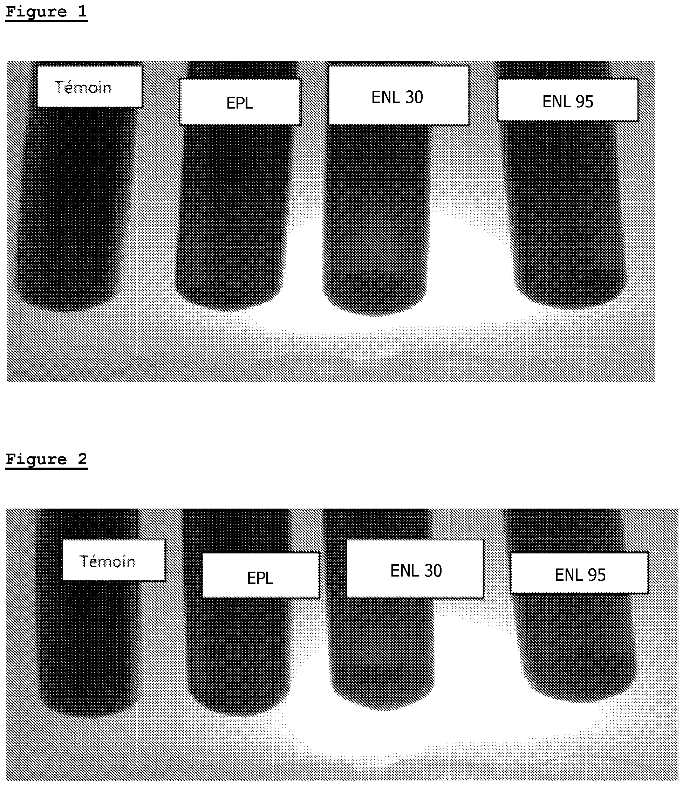

Les photos des figures 1 et 2 illustrent les résultats, après 20 heures en chambre froide, des tests de collage obtenus avec différentes colles (respectivement de gauche à droite : « Témoin », « EPL », « ENL30 » et « ENL95 ») sur les vins « A » (figure 1) et « B » (figure 2).

Les photos des figures 3, 4 et 5 illustrent les résultats, après 20 heures en chambre froide, des tests de collage obtenus avec différentes colles (respectivement de gauche à droite : « Témoin », « EPL », « EPL/ENL30 » et « ENL30 »), sur les vins « BRUT » (figure 3), « A » (figure 4) et « B » (figure 5).

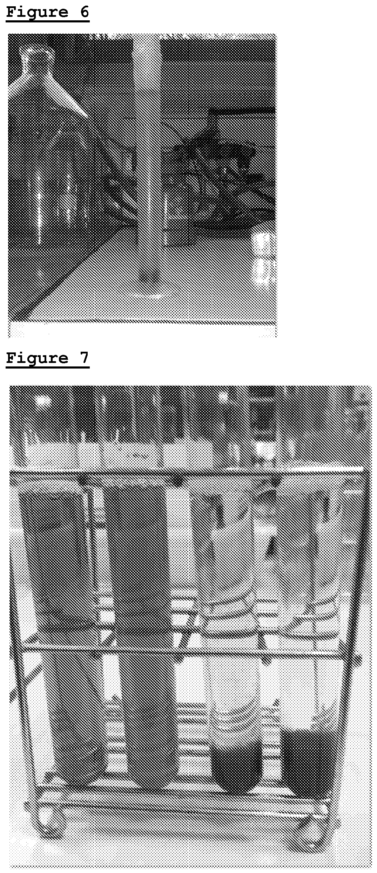

La photo de la figure 6 illustre les résultats du test de collage sur l'ice tea, et plus particulièrement les résultats de la précipitation des tanins de l'ice tea, obtenus après 18 heures à température ambiante avec la colle ENL30 à une concentration de 0,1 g/l.

Les photos de la figure 7 illustrent les résultats des tests de collage sur l'ice tea, et plus particulièrement les résultats de précipitation des tanins de l'ice tea, obtenus après 10 heures à une température de 6°C (voir respectivement de gauche à droite : « Témoin » (ice tea seul), « EPL » à une concentration de 1 g/l, « ENL30 » à une concentration de 0,3 g/l et « ENL30 » à une concentration de 1 g/l).

### EXEMPLES 1 ET 2 : TESTS DE COLLAGE DE VINS

Les vins testés dans les exemples 1 et 2 ci-après sont :
- un vin « brut » (le plus trouble) ;
- un vin « A » ;
- un vin « B ».

Les produits (colles) testé(e)s se trouvent sous forme de poudre et sont :
- un « extrait protéique de levure », représenté par « EPL », qui sert de témoin (cet EPL comprend au total environ 13 % de nucléotides et est décrit dans « Revue des œnologues, N° 120 ; pages 47-50, 2006 ») ;
- un extrait nucléique de levure utilisé dans l'invention comprenant 30% en poids d'acide ribonucléique, représenté par « ENL30 » ;
- un extrait nucléique de levure utilisé dans l'invention comprenant 95% en poids d'acide ribonucléique, représenté par « ENL95 » ;
- un échantillon constitué de 50% d'EPL et de 50% d'ENL30, représenté par « EPL/ENL30 »).

### Exemple 1 : Tests en tube

**1)** Une première série de tests de collage en vin est effectuée avec les colles suivantes : « EPL », « ENL30 » et « ENL95 », et sur les vins suivants « A » (figure 1) et « B » (figure 2).

Les colles (sous forme de poudre) sont incorporées à la dose maximale d'utilisation recommandée pour les vins rouges ou les moûts, à savoir 30 g de colle par hectolitre (hL) de vin/moût.

Les poudres sont préalablement mises en suspension diluée dans 10 fois leur poids d'eau avant incorporation dans le vin. Ainsi, un gramme (1 g) de colle est dilué dans dix millilitres (10 ml) d'eau distillée.

Une quantité de trente microlitres (30 µl) de chaque colle ainsi diluée est incorporée dans un tube à essai (sauf le tube témoin) comprenant 10 ml du vin/moût testé.

L'objectif est de démontrer les différences d'efficacité de collage des différentes colles utilisées à la même concentration et sur deux échantillons différents de vin.

Les résultats obtenus après un séjour de 20 heures des tubes en chambre froide (environ + 4°C) sont illustrés aux figures 1 (pour le vin A) et 2 (pour le vin B).

Plus le culot de décantation observé dans le tube à essai est important (i.e. plus la décantation est importante), plus le collage est satisfaisant.

Les photos des figures 1 et 2 montrent que dans les deux cas le témoin ne présente pas de culot car il n'y a pas de décantation naturelle.

Par contre, dans les tubes comprenant les colles de l'invention (ENL30 et ENL95), on observe un culot de décantation beaucoup plus important (quel que soit le vin testé) que dans le tube comprenant la colle de l'art antérieur (EPL), ce qui permet de mettre en évidence un meilleur collage des vins avec les colles de l'invention comparé à la colle de l'art antérieur.

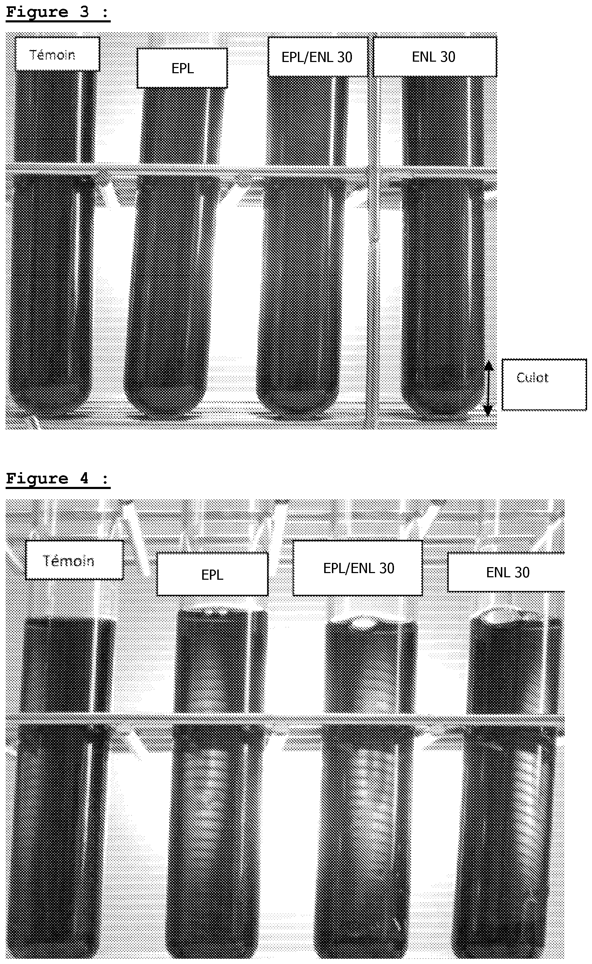

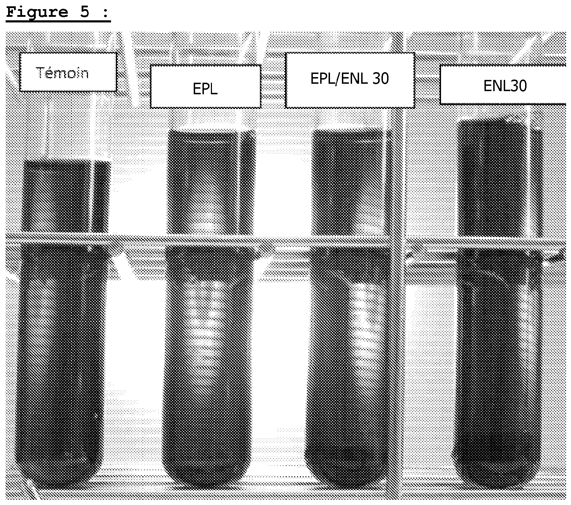

2) Une deuxième série de tests de collage en vin est effectuée avec les colles suivantes : « EPL », « EPL/ ENL30 » et « ENL30 », et sur les vins « brut » (figure 3), « A » (figure 4) et « B » (figure 5).

Les colles sont préparées à la même concentration (30 g/hl) que décrit dans le paragraphe 1) ci-dessus.

Les résultats obtenus après un séjour de 20 heures des tubes en chambre froide (environ + 4°C) sont illustrés aux figures 3 (vin brut), 4 (vin A) et 5 (vin B).

Les photos des figures 3 à 5 montrent que dans les trois cas le témoin ne présente pas de culot (pas de décantation naturelle).

Le plus grand culot de décantation est observé avec la colle de l'invention « ENL30 », quel que soit le vin testé (fig. 3 à 5) .

On observe encore un culot de décantation plus important avec le mélange de colle « EPL/ENL30 » qu'avec la colle de l'art antérieur « EPL », quel que soit le vin testé (fig. 3 à 5).

Ainsi, lorsque l'on prépare une colle comprenant un mélange d'une colle utilisée dans l'invention et de l'art antérieur,
ce mélange permet d'améliorer le collage obtenu avec la colle de l'art antérieur seule, non mélangée à la colle utilisée dans l'invention.

### 3) Conclusion :

Les tests ci-dessus démontrent que les extraits nucléiques de levure utilisés dans l'invention sont des colles
nettement plus efficaces pour les vins que la colle naturelle EPL de l'art antérieur.

Par ailleurs, l'extrait nucléique de levure utilisé dans l'invention présente l'avantage important d'être plus simple à produire que l'EPL et de ne pas nécessiter d'installations complexes.

### Exemple 2 : Tests en cuve spectro

Des tests de turbidité ont été réalisés directement en cuve spectro.

Pour rappel, la turbidité, exprimée en « NTU » (« Nephelometric Turbidity Unit »), désigne la teneur d'un fluide en matières qui le troublent.

Les colles de l'invention « ENL30 » et « ENL95 » sont préparées à trois concentrations différentes : 10 g/hl, 20 g/hl et 30 g/hl, à l'aide d'une dilution préalable de chaque colle dans de l'eau distillée (voir exemple précédent), puis ajout dans le vin « B ».

La turbidité initiale du vin « B » est de 127 NTU.

La mesure de la turbidité dudit vin dans lequel ont été ajoutées les colles de l'invention à différentes concentrations est effectuée au bout de 48 heures en chambre froide (+ 4°C).

Les résultats sont donnés dans le tableau 1 ci-dessous.

**Tableau 1**

| | Vin B | Colle 10 g/hL | Colle 20 g/hL | Colle 30 g/hL |
|---|---|---|---|---|
| ENL95 | 127 NTU | 9,9 NTU | 10,2 NTU | 8,8 NTU |
| ENL30 | 127 NTU | 10,2 NTU | 11 NTU | 10,7 NTU |

L'abaissement de la turbidité est significatif, pour chacune des deux colles, et quelles que soient leur concentrations. Les colles de l'invention « ENL95 » et « ENL30 » sont chacune déjà très actives à une concentration de 10 g/hL (baisse de la turbidité d'un facteur de 12).

On note une clarification quasi-identique des surnageants au bout de 48 heures, quelle que soit la concentration des colles de l'invention.

Les extraits de levure utilisés dans de l'invention, riches en ARN,
sont donc très efficaces comme colles dans le domaine du vin.

### EXEMPLE 3 : TESTS DE COLLAGE DE THE

Le thé testé dans cet exemple est l'ice tea. L'objectif de cet exemple est de démontrer d'une part la précipitation des tanins du thé (qui se retrouvent dans le culot de décantation) et d'autre part le changement de couleur du thé.

La colle testée se trouve sous forme de poudre et est l'ENL30.

Trois échantillons de concentrations différentes sont préparés, à savoir 0,1 g d'ENL30 par litre de thé et 0,3 g d'ENL30 par litre de thé et 1 g d'ENL30 par litre de thé.

La poudre est préalablement diluée dans 10 fois son poids d'eau. Ainsi, un gramme de poudre est dilué dans 10 ml d'eau distillée.

La poudre est totalement soluble dans l'eau, c'est donc une solution limpide que l'on ajoute dans le thé.

### 1) Test à température ambiante

L'ENL30 est introduit dans un tube comprenant de l'ice tea (concentration préparée = 0,1 g/l). Les résultats obtenus après 18 heures à température ambiante sont illustrés à la figure 6.

On observe la formation d'un culot de décantation et une légère décoloration du thé.

Le culot de décantation est prélevé à l'aide d'une pipette et est goûté par 3 personnes, dont un aromaticien.

La sensation perçue par chacun des testeurs est de l'astringence.

La sensation d'astringence étant apportée par les tanins, on peut en conclure une bonne précipitation de ces derniers dans le culot de décantation.

### 2) Test à 6°C

Les quatre échantillons suivants sont testés :
- ice tea seul (témoin),
- ice tea dans lequel a été introduit EPL à une concentration de 1 g/l,
- ice tea dans lequel a été introduit ENL 30 à une concentration de 0,3 g/l,
- ice tea dans lequel a été introduit ENL 30 à une concentration de 1 g/l.

Les résultats obtenus après 10 heures à 6°C sont illustrés à la figure 7.

Les 4 échantillons ci-dessus décrits sont respectivement représentés de gauche à droite de la figure 7 (ainsi l'échantillon tout à gauche est le témoin, celui d'à côté est celui dans lequel a été introduit EPL etc.).

Pour l'ice tea seul, aucune décantation ni décoloration n'est observée.

Pour l'ice tea dans lequel a été introduit l'EPL (1 g/l), une décantation et une légère décoloration sont observées au bout de 2 à 3 mois (pour rappel la figure 7 illustre les résultats au bout de 10 heures, donc pas de décantation ni de décoloration observées), tandis qu'avec l'ice tea dans lequel a été introduit l'ENL (à 0,3 et 1 g/l) une nette décantation et décoloration sont observées au bout de 3 à 6 heures.

Le culot de décantation a été prélevé à l'aide d'une pipette et a été goûté par les 3 testeurs mentionnés au point 1 ci-dessus.

La sensation perçue par chaque testeur au niveau de l'astringence est évaluée dans le tableau 2 ci-dessous.

**Tableau 2 :**

| | **Ice tea tel quel** | **Ice tea + 1 g/L EPL** | **Ice tea + 0,3 g/L ENL** | **Ice tea** + **1 g/L ENL** |
|---|---|---|---|---|
| **Décantation et décoloration** | Aucune | 2-3 mois | 3-6 heures | 3-6 heures |
| **Température** | 6°C | 6°C | 6°C | 6°C |
| **Dégustation du culot de précipitation : Sensation de l'astringence** | 0 | 3 | 3 | 5 |

La sensation d'astringence est un peu plus élevée pour le culot de décantation du tube dans lequel a été ajouté l'ENL30 à une concentration de 0,3 g/l comparée à celle ressentie par le testeur pour le culot de décantation du tube dans lequel a été ajouté l'ENL30 à une concentration de 1 g/l.

### Conclusion :

Au bout de 10 heures, aucune différence n'est observée entre l'échantillon témoin (ice tea seul) et celui dans lequel a été ajouté de l'EPL à une concentration de 1 g/l. L'addition d'EPL dans l'ice tea ne permet pas de conduire à la précipitation des tanins du thé et/ou à la précipitation des pigments du thé.

Par contre, l'addition d'ENL30 à des concentrations telles que 0,3 g/l ou 1 g/l est suffisante pour induire la précipitation des tanins du thé et des pigments du thé, et ce dès 3 à 6 heures après l'ajout d'ENL. La décoloration de l'ice tea est nette pour les tubes dans lesquels ont été ajoutés ENL30, quelle que soit la concentration de l'ENL.

L'addition d'ENL30 à une concentration de 1 g/l dans l'ice tea conduit à un culot de décantation plus important que l'addition d'ENL30 à une concentration de 0,3 g/l.

Par ailleurs, la sensation au niveau de l'astringence est plus élevée pour le culot de décantation du tube dans lequel a été ajouté ENL à 1 g/l.

La sensation d'astringence étant apportée par les tanins, on peut en conclure une bonne précipitation de ces derniers dans le culot de décantation.

## Revendications

1. Méthode de précipitation des particules visibles ou invisibles présentes en suspension dans un moût ou un liquide, ladite méthode comprenant :
l'introduction d'un extrait de levure comprenant une quantité en poids d'acides nucléiques d'au moins 10 %, de préférence d'au moins 15%, plus préférentiellement de 20 à 95% et encore plus préférentiellement de 30 à 48%, par rapport au poids total dudit extrait, lesdits acides nucléiques étant formés de fragments d'acide ribonucléique (ARN),
dans ledit moût ou liquide, ledit moût ou liquide étant choisi parmi le vin, le thé, la bière ou les jus de fruits,
la précipitation dudit extrait de levure avec les particules visibles ou invisibles en suspension dans ledit moût ou liquide conduisant au collage dudit moût ou liquide.

2. Méthode selon la revendication 1, dans laquelle chacun desdits fragments d'ARN de l'extrait présente une masse moléculaire moyenne supérieure à 10 kDa, de préférence allant de 30 à 110 kDa.

3. Méthode selon la revendication 1 ou la revendication 2, dans laquelle l'extrait comprend en outre des matières minérales, des saccharides tels que des glucanes et des mannanes, et des acides aminés libres.

4. Méthode selon l'une quelconque des revendications 1 à 3, dans laquelle l'extrait se présente sous la forme d'une poudre ou d'un liquide plus ou moins concentré, et de préférence sous forme d'une poudre.

5. Méthode selon l'une quelconque des revendications 1 à 4, dans laquelle la levure de l'extrait est choisie dans le groupe comprenant *Saccharomyces, Kluyveromyces, Torula, Candida,* et est de préférence *Saccharomyces,* et avantageusement *Saccharomyces cerevisiae.*

6. Méthode selon l'une quelconque des revendications 1 à 5, pour le collage des vins.

7. Méthode selon la revendication 6, dans laquelle l'extrait de levure se trouve sous forme de poudre et est utilisé à une teneur allant de 0,1 g (gramme) à 50 g par hectolitre (hl) de vin, et de préférence de 5 à 30 g/hl de vin.

8. Méthode selon l'une quelconque des revendications 1 à 5, pour le collage du thé, et en particulier pour la précipitation des tanins et/ou pigments du thé.

9. Méthode selon la revendication 8, dans laquelle l'extrait de levure se trouve sous forme de poudre et est utilisé à une teneur allant de 0,01 g (gramme) à 5 g par litre (1) de thé, et de préférence de 0,05 à 1 g/l de thé.

## Patentansprüche

1. Verfahren zum Ausfällen von sichtbaren oder unsichtbaren Partikeln die in Suspension in einem Most oder einer Flüssigkeit vorliegen, wobei das Verfahren umfasst:
Einbringen eines Hefeextrakts, der eine Gewichtsmenge an Nukleinsäuren von mindestens 10 %, vorzugsweise mindestens 15 %, stärker bevorzugt von 20 bis 95 % und noch stärker bevorzugt von 30 bis 48 %, bezogen auf das Gesamtgewicht des Extrakts, umfasst, wobei die Nukleinsäuren aus Ribonukleinsäure (RNA)-Fragmenten gebildet sind, in den Most oder die Flüssigkeit,
wobei der Most oder die Flüssigkeit aus Wein, Tee, Bier oder Fruchtsäften ausgewählt ist,
wobei der Hefeextrakt mit den in dem Most oder der Flüssigkeit suspendierten sichtbaren oder unsichtbaren Partikeln, bei der Klärung des Mosts oder der Flüssigkeit ausgefällt wird.

2. Verfahren nach Anspruch 1, wobei jedes der RNA-Fragmente des Extrakts ein durchschnittliches Molekulargewicht von mehr als 10 kDa, vorzugsweise im Bereich von 30 bis 110 kDa, aufweist.

3. Verfahren nach Anspruch 1 oder Anspruch 2, wobei der Extrakt weiterhin Mineralien, Saccharide, wie Glucane und Mannane, und freie Aminosäuren enthält.

4. Verfahren nach einem der Ansprüche 1 bis 3, wobei der Extrakt in Form eines Pulvers oder einer mehr oder weniger konzentrierten Flüssigkeit, vorzugsweise in Form eines Pulvers, vorliegt.

5. Verfahren nach einem der Ansprüche 1 bis 4, wobei die Hefe des Extrakts ausgewählt ist aus der Gruppe bestehend aus *Saccharomyces, Kluyveromyces, Torula, Candida* und vorzugsweise *Saccharomyces* und vorteilhafterweise *Saccharomyces cerevisiae* ist.

6. Verfahren nach einem der Ansprüche 1 bis 5 zur Klärung von Weinen.

7. Verfahren nach Anspruch 6, wobei der Hefeextrakt in Pulverform vorliegt und mit einem Gehalt von 0,1 g (Gramm) bis 50 g pro Hektoliter (hl) Wein, vorzugsweise von 5 bis 30 g/hl Wein, eingesetzt wird.

8. Verfahren nach einem der Ansprüche 1 bis 5 zum Klären von Tee, insbesondere zum Ausfällen von Tanninen und/oder Pigmenten aus dem Tee.

9. Verfahren nach Anspruch 8, wobei der Hefeextrakt in Pulverform vorliegt und mit einem Gehalt von 0,01 g (Gramm) bis 5 g pro Liter (I) Tee, vorzugsweise von 0,05 bis 1 g/l Tee, eingesetzt wird.

## Claims

1. A method for precipitating visible or invisible particles present in suspension in a must or a liquid, said method comprising:
the introduction of a yeast extract comprising an amount by weight of nucleic acids of at least 10%, preferably of at least 15%, more preferentially of 20% to 95% and even more preferentially of 30% to 48%, relative to the total weight of said extract, said nucleic acids being formed from fragments of ribonucleic acid (RNA),
in said must or liquid, said must or liquid being chosen from wine, tea, beer or fruit juices,
the precipitation of said yeast extract with the visible or invisible particles in suspension in said must or liquid leading to the clarifying of said must or liquid.

2. Method as claimed in claim 1, wherein each of said fragments of RNA of the extract has an average molecular weight of greater than 10 kDa, preferably ranging from 30 to 110 kDa.

3. Method as claimed in claim 1 or claim 2, wherein the extract also comprises mineral materials, saccharides such as glucans and mannans, and free amino acids.

4. Method as claimed in any one of claims 1 to 3, wherein the extract is in the form of a powder or of a more or less concentrated liquid, and preferably in the form of a powder.

5. Method according to any one of claims 1 to 4, wherein the yeast of the extract is chosen from the group comprising *Saccharomyces, Kluyveromyces, Torula* and *Candida,* and is preferably *Saccharomyces,* and advantageously *Saccharomyces cerevisiae.*

6. Method according to any one of claims 1 to 5, for clarifying wines.

7. Method according to claim 6, wherein the yeast extract is in the form of a powder and is used at a content ranging from 0.1 g (gram) to 50 g per hectoliter (hl) of wine, and preferably from 5 to 30 g/hl of wine.

8. Method according to claims 1 to 5, for clarifying tea, and in particular for precipitating the tannins and/or pigments from tea.

9. Method according to claim 8, wherein the yeast extract is in the form of a powder and is used at a content ranging from 0.01 g (gram) to 5 g per liter (1) of tea, and preferably from 0.05 to 1 g/l of tea.
